# EUROPEAN PATENT SPECIFICATION

(11) **EP 1 199 069 B1**
(45) Date of publication and mention of the grant of the patent: **04.10.2006**
(21) Application number: 01308625.1
(22) Date of filing: 09.10.2001
(51) Int. Cl.: A61K 31/00, A61K 31/40, A61K 31/55, A61K 31/453, A61K 31/138, A61K 31/4535, A61K 31/404, A61K 31/4025, A61K 31/4453, A61K 31/439, A61P 15/02

(54) **Use of an estrogen agonist/antagonist for improving or maintaining urogenital health**
Verwendung eines Östrogen Agonist/Antagonisten zur Verbesserung oder zur Bewahrung der urogenitalen Gesundheit
Utilisation d'un agoniste/antagonist estrogènique pour améliorer ou maintenir la santé urogénitale

(30) Priority: 16.10.2000 US 240789 P
(43) Date of publication of application: 24.04.2002
(73) Proprietor: Pfizer Products Inc., Groton, Connecticut 06340 (US)
(72) Inventor: Day, Wesley Warren, Groton, Connecticut 06340 (US); Lee, Andrew George, Groton, Connecticut 06340 (US); Thompson, David Duane, Groton, Connecticut 06340 (US)
(74) Representative: Cosway, Sarah Michelle

(56) References cited:
- EP-A- 0 792 641
- EP-A- 1 149 579
- WO-A-01/54699

## Description

### FIELD OF THE INVENTION

The present invention relates to the use of an estrogen agonist/antagonist for the manufacture of a medicament for treating vaginitis; treating bacterial vaginitis; treating a vaginal yeast or bacterial infection; treating vulvar atrophy; treating urethrocele, cystocele, rectocele, or enterocele prolapse; or treating undesired urinary frequency or urgency; in a female patient.

The invention also relates to the non-medical use of an estrogen agonist/antagonist for increasing the frequency or intensity of orgasms in a female patient.

In postmenopausal women, conditions such as urinary and vaginal infections; incontinence; and vaginal dryness can be treated.

### BACKGROUND OF THE INVENTION

Menopause occurs naturally in women at an average age of 50 to 51 years in the United States. As ovaries age, response to pituitary gonadotropins (follicle-stimulating hormone [FSH] and luteinizing hormone [LH]) decreases, initially resulting in shorter follicular phases (thus, shorter menstrual cycles), fewer ovulations, decreased progesterone production, and more irregularity in menstrual cycles. Eventually, the follicle fails to respond and does not produce estrogen. The transitional phase, during which a woman passes out of the reproductive stage, begins before menopause. It is termed the climacteric or perimenopause, although many persons refer to it as menopause.

Premature menopause refers to ovarian failure of unknown cause that occurs before age 40. It may be associated with smoking, living at high altitude, or poor nutritional status. Artificial menopause may result from oophorectomy, chemotherapy, radiation of the pelvis, or any process that impairs ovarian blood supply or ovarian function.

Symptoms of the climacteric range from nonexistent to severe. Hot flushes (flashes) and sweating secondary to vasomotor instability affect 75% of women going through the perimenopausal period. Most have hot flushes for more than 1 year, and 25 to 50% for more than 5 years. The woman feels warm or hot and may perspire, sometimes profusely. The skin, especially of the head and neck, becomes red and warm. The flush, which may last from 30 seconds to 5 minutes, may be followed by chills. Vasomotor symptoms of the hot flush coincide with the onset of LH pulses, but not every increase in LH is associated with a hot flush, suggesting that hypothalamic control of LH pulses is independent of that of flushes. This independence is confirmed by the occurrence of hot flushes in women who have had pituitary failure and do not secrete LH and/or FSH.

The large reduction in estrogen leads to profound changes in the lower genital tract; e.g., the vaginal mucosa and vulvar skin become thinner, the normal bacterial flora changes, and the labia minora, clitoris, uterus, and ovaries decrease in size. Inflammation of the vaginal mucosa (atrophic vaginitis) can cause the mucosa to have a strawberry appearance and can lead to urinary frequency and urgency, vaginal dryness, and dyspareunia. Women tend to lose pelvic muscle tone and to develop urinary incontinence, cystitis, and vaginitis.

Normal vaginal secretion is composed of vulvar secretions from sebaceous, sweat, Bartholin, and Skene glans; transudate from the vaginal wall; exfoliated vaginal and cervical cells; cervical mucous; endometrial and oviductal fluids; and microorganisms and their metabolic products. The type and amount of exfoliated cells, cervical mucous, and upper genital tract fluids are determined by the biochemical processes that are influenced by hormone levels (Huggins, G.R. and Preti, G. Clin Obstet Gynecol, 1981;24:355-377). The vaginal desquamative tissue is made up of vaginal epithelial cells that are responsive to varying amounts of estrogen and progesterone. Superficial cells, the predominant cell type in women of reproductive age, predominate when estrogen stimulation is present. Intermediate cells predominate during the luteal phase because of progestogenic stimulation. Parabasal cells predominate in the absence of either hormone, a condition that may be found in postmenopausal women who are not receiving hormone replacement therapy.

The normal vaginal flora is predominately aerobic, with an average of six different species of bacteria, the most common of which is hydrogen peroxide producing lactobacilli. The microbiology of the vagina is determined by factors that affect the ability of bacteria to survive. These factors include vaginal pH and the availability of glucose for bacterial metabolism. The premenopausal vagina is acidic, usually below a pH of 4.5. The environment is maintained by the presence of estrogen, which stimulates vaginal epithelial cells to produce glycogen, which can then be converted by lactobacilli to lactic acid. Lack of estrogenic stimulation of the vagina results in reduction in available glycogen and an increase in vaginal pH resulting in a change in vaginal flora.

Bacterial vaginosis (BV) has previously been referred to as nonspecific vaginitis or *Gardnerella vaginitis.* It is an alteration of normal vaginal bacterial flora that results in the loss of hydrogen peroxide-producing lactobacilli and an overgrowth of predominately anaerobic bacteria (Eschenbach, D.A., *et al., J Clin Microbiol,* 1989;**27**:251-256; Spiegel, C.A., *et al., N Engl J Med,* 1980;**303**:601-607). The most common form of vaginitis in the United States is BV. Anaerobic bacteria can be found in less than 1 % of the flora of normal women. In women with BV, however, the concentration of anaerobes, as well as *Gardnerella vaginalis* and *Mycoplasma hominis,* is 100 to 1000 times higher than in normal women. Lactobacilli are usually absent.

Numerous studies have shown an association of BV with significant adverse sequelae. Women with BV have an increased risk of pelvic inflammatory disease (PID) (Eschenbach, D.A., *et al., Am J Obstet Gynecol,* 1988;**158**:819-828), postoperative cuff infections after hysterectomy (Soper, D.E. *et al., Am J Obstet Gynecol,* 1990;**163**:1016-1023), and abnormal cervical cytology (Platz-Christensen, J.J., *et al., Acta Obstet Gynecol Scand,* 1994;**73**:586-588).

Urinary tract infection in women may involve acute cystitis, recurrent cystitis, and urethritis. Women with acute cystitis generally have an abrupt onset of multiple, severe urinary tract symptoms including dysuria, frequency, and urgency associated with suprapubic or low back pain. Suprapubic tenderness may be noted on physical examination. Urinalysis reveals pyuria and sometimes hematuria. About 20% of premenopausal women with an initial episode of cystitis will have recurrent infections. More that 90% of these recurrences are caused by exogenous reinfection. Postmenopausal women may also have frequent reinfections. Hormonal replacement therapy or topically applied estrogen cream along with antimicrobial prophylaxis has been used in treating these patients. Women with dysuria caused by urethritis have a more gradual onset of mild symptoms, which may be associated with abnormal vaginal discharge or bleeding related to concurrent cervicitis. Patients may also experience lower abdominal pain. Physical examination may reveal the presence of mucopurulent cervicitis.

Vaginal dryness in postmenopausal women is presumed to be caused by vaginal atrophy due to decreased estrogenic stimulation. When estrogen levels are low or absent, vascularity of the vagina is reduced and vaginal epithelium is thinned. The decrease in vascularity and vaginal epithelium results in less transudation and vaginal moisture.

The genital and urinary tracts are intimately associated anatomically and embryologically from the earliest stages of their development. The bladder is located directly above the anterior vaginal wall and the urethra is fused to it. Both of these structures, as well as structures of the pelvic floor, are placed at risk during pregnancy and childbirth. In postmenopausal women, changes in the pelvic floor may occur due to changes in hormonal status that consequently result in incontinence, prolapse, and other disorders.

Each organ system in the pelvic floor, urinary, genital, and intestinal, traverses the pelvis and exits through its own orifice. Thus, these systems are intricately related in function and anatomic support (Wall, L.L. and DeLancey, J.O.L., *Perspect Biol Med,* 1991;**34**:486-496). Disorders of each of these components can necessarily have an impact on the functioning of the surrounding structures and the functional anatomy of the pelvic floor. The striated muscles of the pelvic floor, in combination with their fascial attachments, work together across the entire pelvis to prevent pelvic organ displacement, to maintain continence, and to control expulsive activities. Due to these complex interrelationships, each disturbance of pelvic support may be linked to problems in other organ systems. Such is the case with incontinence disorders.

The nomenclature of the pelvic muscles has been subject to debate. The levator ani muscle (the broad general term for the muscles of the pelvic floor) has been described as consisting of a diaphragmatic portion (iliococcygeus) and the more important "pubovisceral" portion (Lawson, J.O. *Ann R Coll Sur Engl* 1974;**54**:244-252). The iliococcygeus or "diaphragmatic" portion of the levator ani consists of a thin muscular sheet that arises from the pelvic sidewall on either side of the arcus tendinous and ischial spine and inserts into a midline raphe behind the rectum. The pubovisceral ("pubococcygeus") portion of the levator ani muscle consists of a thick U-shaped band of muscle arising from the pubic bone and attaching to the lateral walls of the vagina and rectum. Therefore, the rectum is supported by a muscular sling that pulls it toward the pubic bones when the muscles contract. The muscular band is often called the puborectalis or the pubococcygeus muscle or the pubovisceral muscle. When the pubovisceral contracts, it pulls the rectum, vagina, and urethra anteriorly toward the pubic bone and constricts the lumen of these pelvic organs. It is this contractile property that is so important in maintaining urinary and fecal continence and in providing support of the genital organs (vagina, cervix, uterus) that lie upon and are supported by the levator plate.

Connective tissue is composed primarily of elastin and collagen fibers in a polysaccharide ground substance. The composition of connective tissue is not constant but varies in different sites throughout the body. Connective tissue forms capsules to help maintain the structural integrity of the organs. If connective tissue fails, muscular support will be weak. Connective tissue is not static but instead is a dynamic tissue that undergoes constant turnover and remodeling. Hormonal changes have significant effects on collagen which is thus related to aging and the postmenopausal state (Brincat, M. *et al., Obstet Gynecol,* 1987;**70:**123-127; Castelo-Branco, C. *et al., Maturitas,* 1992;**15:**113-119). Connective tissue abnormalities are a significant factor contributing to prolapse and related conditions such as urinary and fecal incontinence.

In premenopausal women, 17β-estradiol produced by the ovaries is the chief active circulating estrogen. Serum estradiol concentrations are low in preadolescent girls and increase at menarche. In women, they range from about 100 pg per milliliter (367 pmol per liter) in the follicular phase to about 600 pg per milliliter (2200 pmol per liter) at the time of ovulation. They may rise to nearly 20,000 pg per milliliter (70,000 pmol per liter) during pregnancy. After menopause, serum estradiol concentrations fall to values similar to or lower than those in men of similar age (5 to 20 pg per milliliter [18 to 74 pmol per liter]) (Yen, S.S.C. and Jaffe, R.B., eds. *Reproductive Endocrinology: Physiology, Pathophysiology and Clinical Management,* 3rd ed. Philadelphia: W.B. Saunders, (1991)).

The effects of estrogen on urogenital health and tone are generally positive, however, some of the non-urogenital effects of estrogen, such as increased risk of breast cancer or the occurrence of blood clots may offset its beneficial effects.

Breast cancer is typically or often a hormone-dependent disease. Women without functioning ovaries who never receive estrogen replacement rarely develop breast cancer. The female-to-male ratio for the disease is about 150 to 1. Findings indicate that hormones play a critical role as promoters of the disease. For most epithelial malignancies, a log-log plot of incidence versus age shows a straight-line increase with every year of life. A similar plot for breast cancer shows the same straight line increase, but with a decrease in slope beginning at the age of menopause. The three dates in a woman's life that have a major impact on breast cancer incidence are age of menarche, age at first full-term pregnancy, and age of menopause. Women who experience menarche at age 16 have only 50 to 60 percent of the lifetime breast cancer risk of women who experience menarche at age 12. Similarly, menopause occurring 10 years before the median age (52 years), whether natural or surgically induced, reduces lifetime breast cancer risk by about 35 percent. Compared with nulliparous women, women who have a first full-term pregnancy by age 18 have 30 to 40 percent the risk of breast cancer. Thus, length of menstrual life-particularly the fraction occurring before the first full-term pregnancy-is a substantial component of the total risk of breast cancer. This factor can account for 70 to 80 percent of the variation in breast cancer frequency in different countries.

International variation has provided some of the most important dues on hormonal carcinogenesis. A woman living to age 80 in North America has 1 chance in 9 of developing invasive breast cancer. Asian women have one-fifth to one-tenth the risk of breast cancer of women in North America or Western Europe. Asian women have substantially lower concentrations of estrogens and progesterone. These differences cannot be explained on a genetic basis, because Asian women living in a Western environment have a risk identical to that of their Western counterparts. These women also differ markedly in height and weight from Asian women in Asia; height and weight are critical regulators of age of menarche and have substantial effects on plasma concentrations of estrogens. (Lippman, M.E., *Breast* Cancer, Chapter 91, in *Harrison's Principles of Internal Medicine,* 14th ed., 1998). Labrie (WO 01 /54699, Endorecherche Inc) discusses the use of a combination of a Selective Estrogen Receptor Modulator (SERM) and an estrogen for the reduction or elimination of menopausal symptoms. Lee *et al.* (EP-A-1 149 579, Pfizer Products Inc) discuss the use of an estrogen agonist/antagonist for the treatment of female sexual dysfunction. MacClean and Thompson (EP-A-0 792 641, Pfizer Inc) discuss the use of an estrogen agonist/antagonist for the treatment of a number of pathological conditions.

### SUMMARY OF THE INVENTION

The present invention provides the use of an estrogen agonist/antagonist for the manufacture of a medicament for treating vaginitis; treating bacterial vaginitis; treating a vaginal yeast or bacterial infection; treating vulvar atrophy; treating urethrocele, cystocele, rectocele, or enterocele prolapse; or treating undesired frequency or urgency; in a female patient; wherein the estrogen agonist/antagonist is a compound of formula (I).

The present invention also provides the non-medical use of an estrogen agonist/antagonist for increasing the frequency or intensity of orgasms in a female patient, wherein the estrogen agonist/antagonist is a compound of formula (I).

Compounds of formula (I) are of the structure given below: 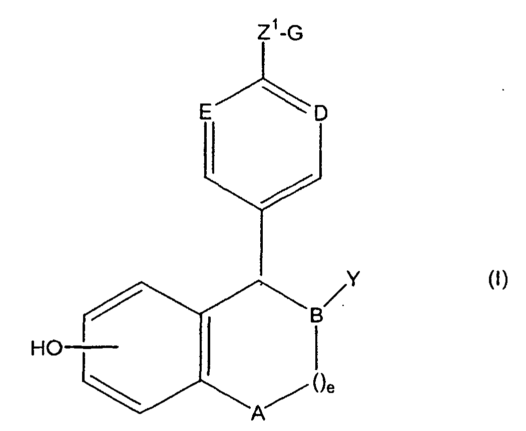 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 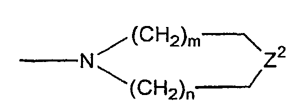 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or 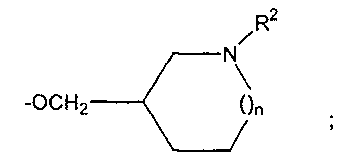
Z¹ and G in combination may be
W is
   (a) -CH₂-;
   (b) -CH=CH-;
   (c) -O-;
   (d) -NR²-;
   (e) -S(O)ₙ-;
   (f) 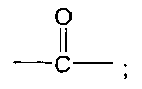
   (g) -CR²(OH)-;
   (h) -CONR²-;
   (i) -NR²CO-;
   (j) 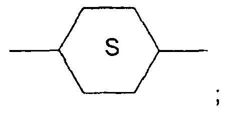 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (l) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, or quaternary ammonium salt thereof.

In another preferred embodiment of the uses the estrogen agonist / antagonist is a compound of formula (IA) 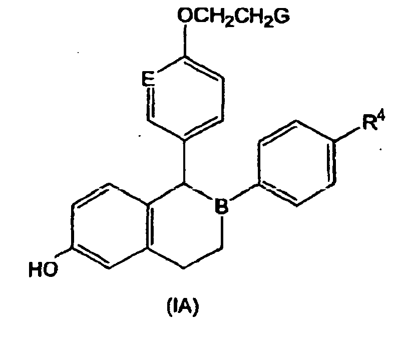 wherein G is 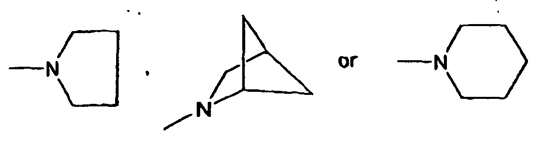 R⁴ is H, OH, F, or Cl; and B and E are independently selected from CH and N or an optical or geometric isomer thereof; or a pharmaceutically acceptable salt, N-oxide, ester, or quaternary ammonium salt thereof.

In a preferred embodiment of the methods, the estrogen agonist / antagonist is (-)-cis-6-phenyl-5-[4-(2-pyrrolidin-1-yl-ethoxy)-phenyl]-5,6,7,8-tetrahydronaphthalene-2-ol or an optical or geometric isomer thereof; a pharmaceutically acceptable salt, N-oxide, ester, or quaternary ammonium salt, thereof.

In another preferred embodiment of the methods, the estrogen agonist / antagonist is (-)-cis-6-phenyl-5-[4-(2-pyrrolidin-1-yl-ethoxy)-phenyl]-5,6,7,8-tetrahydro-naphthalene-2-ol, D-tartrate salt.

In a preferred embodiment of the methods, the patient is a postmenopausal woman.

### DETAILED DESCRIPTION OF THE INVENTION

The present invention relates to the use of an estrogen agonist/antagonist for the manufacture of a medicament for treating vaginitis, treating vaginal yeast or bacterial infections, treating vulvar atrophy, treating urethrocele, cystocele, rectocele, or enterocele prolapse, and treating undesired urinary frequency or urgency.

The present invention further relates to the non-medical use of an estrogen agonist/antagonist for increasing the frequency and intensity of orgasms.

As used herein, the terms "treat", "treatment" and "treating" include preventative (e.g., prophylactic) and palliative treatment of the disease or condition, or amelioration of a symptom of the disease or condition.

The term "incontinence" includes urinary and anal incontinence. When the incontinence is urinary in nature, it is defined as involuntary urine loss that is a social or hygienic problem. Urinary incontinence may be stress incontinence, urge incontinence or mixed incontinence which is stress and urge incontinence occurring together, or it may be unconscious incontinence which occurs without urgency and without conscious recognition of leakage. There may be hesitancy, straining to void, poor stream as indicated by a decreased force of flow of the urinary stream. Intermittent stream may be noted as a "stop and start" pattern of urination. Or there may be incomplete emptying of the bladder or postmicurition dribble that is urine loss occurring just after normal urination has been completed.

A "prolapse" is a downward or forward displacement of one of the pelvic organs from its normal location. Traditionally, prolapse has referred to displacement of the bladder, the uterus, or rectum. Prolapse can also relate to displacement of the vagina. These displacements have usually been graded on a scale of 0-4; the grade increases with increasing severity of the prolapse. A variety of terms are used to describe female genital prolapse that have been fixed in the literature, which terms include:

A "cystocele" is a downward displacement of the bladder.

A "cystourethocele" is a cystocele that includes the urethra as part of the prolapsing organ complex.

A "uterine prolapse" is descent of the uterus and cervix down the vaginal canal toward the vaginal introitus.

A "rectocele" is a protrusion of the rectum into the posterior vaginal lumen.

A "enterocele" is a herniation of the small bowel into the vaginal lumen.

The term "vaginitis" means inflammation of the vagina.

The phrase "undesired urinary frequency or urgency" means that a patient urinates more often than an average of a group of similar patients. Typically, this increased number of urinations makes the patient psychologically uncomfortable and is embarrassing. In addition, a patient can experience an enhanced sense of the need to urinate when compared with a similar group. This heightened sense of needing to urinate can also lead to psychological discomfort and embarrassment.

The term "increasing the frequency or intensity of orgasms" means that a patient experiences more orgasms or experiences or perceives a heightened intensity of orgasms after treatment with the present compounds than without treatment. A way to measure a patient's perception of increased number of orgasms and/or increased intensity of orgasms is to ask the patient. For example, a questionnaire can be used.

Some aspects of vaginal health status can be determined by analysis of vaginal secretions. Normal vaginal secretions are floccular in consistency, white in color, and usually located in the dependent portion of the vagina (posterior fomix). Vaginal secretions can be analyzed by a wet-mount preparation. A sample of vaginal secretions is suspended in 0.4 mL of normal saline in a glass tube, transferred to a slide, covered with a slip, and assessed by microscopy. Some clinicians prefer to prepare slides by suspending secretions in saline placed directly on the slide. Secretions should not be placed directly on the slide without saline because this method causes drying of the vaginal secretions and does not result in a well-suspended preparation. Microscopy of normal vaginal secretions reveals many superficial epithelial cells, a few white blood cells (less than one per epithelial cell), and few, if any, clue cells. Clue cells are superficial vaginal epithelial cells with adherent bacteria, usually *G. vaginalis,* which obliterates the crisp cell border and usually can be visualized microscopically. Potassium hydroxide 10% (KOH) may be added to the slide, or a separate preparation can be made, to examine the secretions for fungal elements. The results should be negative for women with normal vaginal microbiology. Gram stain will reveal that normal superficial epithelial cells appear normal and a predominance of gram positive rods (lactobacilli).

The phrase "adverse effects associated with estrogen" include breast tenderness, bloating, headache, increased blood clotting and menstrual bleeding in women and breast cancer. Estrogen therapy increases the risk of endometrial carcinoma. Women on long-term estrogen therapy may have an increased risk that is not reversed by concurrent administration of progestin (*N Engl J Med* 1995;**332**:1589).

The term "postmenopausal women" is defined to include not only women of advanced age who have passed through menopause, but also women who have been hysterectomized or for some other reason have suppressed estrogen production, such as those who have undergone long-term administration of corticosteroids, suffer from Cushings' syndrome, have gonadal dysgenesis or who have undergone radiation therapy.

"Breast cancer" is defined as a malignant proliferation of epithelial cells lining the ducts or lobules of the breast.

The term "patient" means animals, particularly mammals. Preferred patients are humans, with postmenopausal female humans being the most preferred patients.

An "estrogen agonist / antagonist" is a compound that affects some of the same receptors that estrogen does, but may not affect all, and in some instances, it antagonizes or blocks estrogen. It is also known as a "selective estrogen receptor modulator" (SERM). Estrogen agonists / antagonists may also be referred to as antiestrogens although they have some estrogenic activity at some target tissues. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, *et al., Steroids,* 1973;**22**:707, Capony et *al., Mol Cell Endocrinol,* 1975;**3**:233).

When a woman reaches menopause, changes in the urogenital system can occur. These changes include increased vaginal pH; increased number of vaginal yeast and bacterial infections, which can be exacerbated by increased vaginal pH; increased vaginal dryness and itching; undesired vaginal spasms; vaginitis; vulvar atrophy; various types of prolapse as described herein; and urinary or anal incontinence, which can be the result of a prolaspse. In accordance with the present invention, these conditions can be treated by administering an estrogen agonist / antagonist. By treating these conditions, the overall vaginal health of a patient is maintained or improved.

The estrogen agonists / antagonists of the invention may be administered systemically or locally. For systemic use, the estrogen agonists / antagonists herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from monthly, weekly to once to three or more times daily.

Another method of administering compounds of the present invention includes the use of topical dosage forms. For example, the active agent can be administered to a patient in a cream or ointment that is applied to the skin, particularly the skin of the vagina. Alternatively, the active agent can be delivered using a patch that is applied to the skin. The use of a topical dosage form is particularly useful in treating vaginal dryness, urinary and/or vaginal bacterial or yeast infections and dyspureunia.

Preferred estrogen agonists / antagonists that can be used in the uses of the present invention include the compounds described in U.S. patent number 5,552,412. Those compounds are described by the formula designated herein as formula (I) given below: 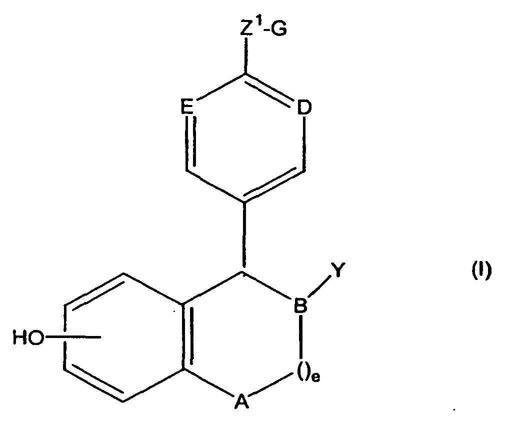 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚ W(CH₂)_{q}-;
   (b) -O(CH₂)ₚ CR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 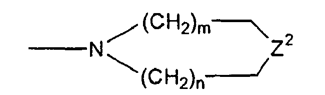 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 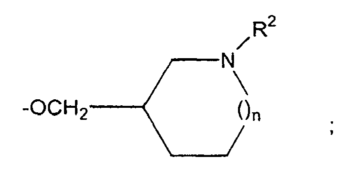
W is
   (a) -CH₂-;
   (b) -CH=CH-;
   (c) -O-;
   (d) -NR²-;
   (e) -S(O)ₙ-;
   (f) 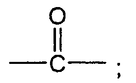
   (g) -CR²(OH)-;
   (h) -CONR²-;
   (i) -NR²CO-;
   (j) 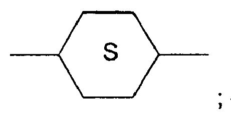 or
   (k) -C=C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (l) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₆ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ In either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
and optical and geometric isomers thereof; and nontoxic pharmacologically acceptable add addition salts. N-oxides, esters, or quaternary ammonium salts thereof.

Additional preferred compounds of the invention also disclosed in U.S. patent number 5,552,412 are described by the formula designated herein as formula (IA): 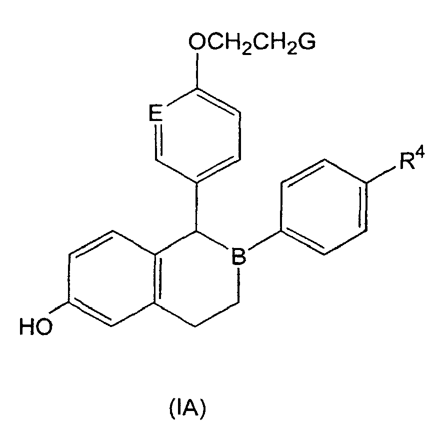 wherein G is 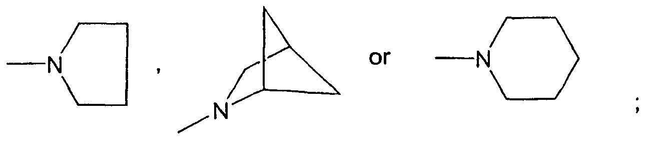 R⁴ is H, OH, F, or Cl; and B and E are independently selected from CH and N, and pharmaceutically acceptable salts thereof.

Especially preferred compounds for the methods of the invention are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydronaphthalene-2-ol;
cis-6-phenyl-5-[4-(2-pyrrolidin-1-yl-ethoxy)-phenyl]-5,6,7,8-tetrahydronaphthalene-2-ol;
cis-1-[6'-pyrrolidinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;
1-(4'-pyrrolidinoethoxyphenyl)-2-phenyl-6-hydroxy-1,2,3,4-tetrahydroisoquinoline; and pharmaceutically acceptable salts thereof. An especially preferred salt of (-)-cis-6-phenyl-5-[4-(2-pyrrolidin-1-yl-ethoxy)-phenyl]-5,6,7,8-tetrahydro-naphthalene-2-ol is the D-tartrate salt.

The syntheses of compounds of formula (I) are set forth in U.S. patent number 5,552,412. agonists / antagonists of this invention may be formed of the compound itself, or of any of its esters, and include the pharmaceutically acceptable salts which are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferable with hydrochloric acid, citric acid, benzoic acid, maleic acid, acetic acid or propionic acid.

The estrogen agonists / antagonists of this invention, as discussed above, can be administered in the form of pharmaceutically acceptable salts. The salts are conveniently formed, as is usual in organic chemistry, by reacting the compound of this invention with a suitable acid, such as has been described above. The salts are quickly formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6,7,8-tetrahydro-naphthalene-2-ol is the D-(-)-tartrate salt. It will also be recognized that it is possible to administer amorphous forms of the estrogen agonists / antagonists.

The expression "pharmaceutically acceptable salts" includes both pharmaceutically acceptable acid addition salts and pharmaceutically acceptable cationic salts. The expression "pharmaceutically-acceptable cationic salts" is intended to define but is not limited to such salts as the alkali metal salts, (e.g. sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

One of ordinary skill in the art will recognize that certain estrogen agonists / antagonists of this invention will contain one or more atoms which may be in a particular stereochemical, tautomeric, or geometric configuration, giving rise to stereoisomers, tautomers and configurational isomers. All such tautomers and isomers and mixtures thereof are included in this invention. Hydrates and solvates of the compounds of this invention are also included.

The subject invention also includes isotopically-labeled estrogen agonists / antagonists, which are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O,¹⁷O, ³¹P, ³²P, ³⁵S, ¹⁸F and ³⁶Cl, respectively. Compounds of the present invention, prodrugs thereof, and pharmaceutically acceptable salts of said compounds and of said prodrugs which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in compound and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Certain ester groups are preferred when a compound of this invention contains an ester. The estrogen agonists / antagonists including the compounds of formula I, or IA, may contain ester groups at various positions as defined herein above, where these groups are represented as -COOR⁹, R⁹ is C₁-C₁₄ alkyl, C₁-C₃ chloroalkyl, C₁-C₃ fluoroalkyl, C₅-C₇ cycloalkyl, phenyl, or phenyl mono- or disubstituted with C₁-C₄ alkyl, C₁-C₄ alkoxy, hydroxy, nitro, chloro, fluoro or tri(chloro or fluoro)methyl.

As used herein, the term "effective amount" means an amount of an estrogen agonist / antagonist or combination of estrogen agonists / antagonists that is capable of treating the described pathological condition. The specific dose of a compound or combination of compounds administered according to this invention will, of course, be determined by the particular circumstances including, for example, the compound or combination administered, the route of administration, and the severity of the pathological condition being treated.

The dose of a compound of this invention to be administered to a patient is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts and other dosage amounts set forth elsewhere in this description and in the appendant claims are for an average human patient having a weight of about 65 kg to about 70 kg. The skilled practitioner will readily be able to determine the dosage amount required for a patient whose weight falls outside the 65 kg to 70 kg range. All doses set forth herein, and in the appendant claims, are daily doses of the free base form of the estrogen agonists / antagonists. Calculation of the dosage amount for other forms of the free base form such as salts or hydrates is easily accomplished by performing a simple ratio relative to the molecular weights of the species involved.

The general range of effective administration rates of the estrogen agonists / antagonists is from about 0.001 mg/day to about 200 mg/day. A preferred rate range is from about 0.010 mg/day to 100 mg/day. Of course, it is often practical to administer the daily dose of compound in portions, at various hours of the day. However, in any given case, the amount of compound administered will depend on such factors as the potency of the specific estrogen agonist / antagonist, the solubility of the compound, the formulation used and the route of administration.
For (-)-cis-6-phenyl-5-[4-(2-pyrrolidin-1-yi-ethoxy)-phenyl]-5,6,7,8-tetrahydronaphthalene-2-ol, L-tartrate salt, a preferred dosage range for humans is about 0.025 mg to about 1 mg per day. A more preferred dosage range is about 0.25 to about 0.5 mg per day.

Methods of formulation are well known in the art and are disclosed, for example, in *Remington's Pharmaceutical Sciences,* Mack Publishing Company, Easton, Pa., 19th Edition (1995). Pharmaceutical compositions for use within the present invention can be in the form of sterile, non-pyrogenic liquid solutions or suspensions, coated capsules, suppositories, lyophilized powders, transdermal patches or other forms known in the art.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances that facilitate the disintegration of a tablet to release a compound when the tablet becomes wet. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavorant and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Topical formulations may be designed to yield delayed and/or prolonged percutaneous absorption of a compound. Even the parenteral preparations may be made long-acting, by dissolving or suspending the compound in oily or emulsified vehicles which allow it to disperse only slowly in the serum.

The present invention may also be provided in a kit for use by a consumer to improve or maintain urogenital health. The kits comprise a) a pharmaceutical composition comprising an estrogen agonist / antagonist and a pharmaceutically acceptable carrier, vehicle or diluent; and b) instructions describing a method of using the pharmaceutical compositions to improve or maintain urogenital health. The instructions may also indicate that the kit is to improve or maintain urogenital health while substantially reducing the concomitant liability of adverse effects associated with estrogen administration.

A "kit" as used in the instant application includes a container for containing the pharmaceutical compositions and may also include divided containers such as a divided bottle or a divided foil packet. The container can be in any conventional shape or form as known in the art which is made of a pharmaceutically acceptable material, for example a paper or cardboard box, a glass or plastic bottle or jar, a resealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle that is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a written memory aid, where the written memory aid is of the type containing information and/or instructions for the physician, pharmacist or patient, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday," ... etc .... "Second Week, Monday, Tuesday,..." etc. Other variations of memory aids will be readily apparent. A "daily dose" can be a single tablet or capsule or several tablets or capsules to be taken on a given day.

Another specific embodiment of a kit is a dispenser designed to dispense the daily doses one at a time. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

It is noted that an estrogen agonist / antagonist can be used in combination with other different estrogen agonists / antagonists to maintain or improve urogenital health. In addition, estrogen agonists /antagonists can be used in combination with estrogen. Estrogen agonists / antagonists can also be used in combination with one or more additional compounds that are therapeutically useful to improve or maintain urogenital health. For example, estrogen agonists / antagonists can be used in combination with compounds that are used to treat urinary incontinence, anal incontinence, vaginal infections, urinary infections, vaginal dryness, vaginal itching, or pelvic floor integrity, including vaginal prolapse. Examples of additional compounds that can be used in combination with an estrogen agonist / antagonist include Detrol® and anti-fungal and anti-bacterial products.

In addition, an estrogen agonist / antagonist can be used in combination with agents that can be used to acidify (i.e., lower pH) the urinary tract and/or vagina. Examples of such agents include potassium acid phoshate and sodium acid phosphate.

An estrogen agonist / antagonist can be used in combination with a cGMP elevator agent to treat the conditions disclosed herein.

Preferred as the cGMP elevator are cGMP phosphdiesterase (PDE) inhibitors. cGMP PDE inhibitors that are selective for cGMP PDEs rather than cyclic adenosine 3',5'-monophosphate phosphodiesterases (cAMP PDEs) and that are selective inhibitors of the cGMP PDEᵥ isoenzyme are particularly preferred. Such particularly preferred cGMP PDE inhibitors are disclosed in US patents 5,250,534; 5,346,901; 5,272,147; and in the international patent application WO 94/28902.

Preferred cGMP PDEᵥ inhibitors include compounds of formula (VII): 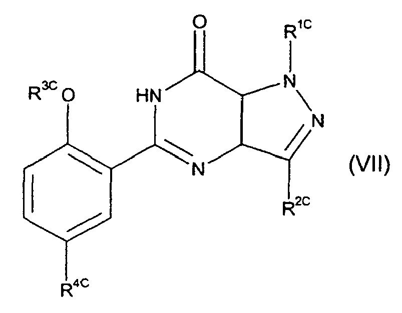 wherein:
R^{1C} is H; C₁-C₃ alkyl; C₁-C₃ perfluoroalkyl; or C₃-C₅ cycloalkyl;
R^{2C} is H; C₁-C₆ alkyl optionally substituted with C₃-C₆ cycloalkyl; C₁-C₃ perfluoroalkyl; or C₃-C₆ cycloalkyl;
R^{3C} is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R^{4C} is C₁-C₄ alkyl optionally substituted with OH, NR^{5C}R^{6C}, CN, CONR^{5C}R^{6C} or CO₂R^{7C}; C₂-C₄ alkenyl optionally substituted with CN, CONR^{5C}R^{6C} or CO₂R^{7C}; C₂-C₄ alkanoyl optionally substituted with NR^{5C}R^{6C}; (hydroxy)C₂-C₄ alkyl optionally substituted with NR^{5C}R^{6C}; (C₂-C₃ alkoxy)C₁-C₂ alkyl optionally substituted with OH or NR^{5C}R^{6C}; CONR^{5C}R^{6C}; CO₂R^{7C}; halo; NR^{5C}R^{6C}; NHSO₂NR^{5C}R^{6C}; NHSO₂R^{8C}; SO₂NR^{9C}R^{10C} or phenyl, pyridyl, pyrimidinyl, imidazolyl, oxazolyl, thiazolyl, thienyl or triazolyl any of which is optionally substituted with methyl;
R^{5C} and R^{6C} are each independently H or C₁-C₄ alkyl, or together with the nitrogen atom to which they are attached form a pyrrolidinyl, piperidino, morpholino, 4-N(R^{11C})-piperazinyl or imidazolyl group wherein said group is optionally substituted with methyl or OH;
R^{7C} is H or C₁-C₄ alkyl;
R^{8C} is C₁-C₃ alkyl optionally substituted with NR^{5C}R^{6C};
R^{9C} and R^{10C} together with the nitrogen atom to which they are attached form a pyrrolidinyl, piperidino, morpholino or 4-N(R^{12C})-piperazinyl group wherein said group is optionally substituted with C₁-C₄ alkyl, C₁-C₃ alkoxy, NR^{13C}R^{14C} or CONR^{13C}R^{14C};
R^{11C} is H; C₁-C₃ alkyl optionally substituted with phenyl; (hydroxy)C₂-C₃ alkyl; or C₁-C₄ alkanoyl;
R^{12C} is H; C₁-C₆ alkyl; (C₁-C₃ alkoxy)C₂-C₆ alkyl; (hydroxy)C₂-C₆ alkyl; (R^{13C}R^{14C}N)C₂-C₆ alkyl; (R^{13C}R^{14C}NOC)C₁-C₆ alkyl; CONR^{13C}R^{14C}; CSNR^{13C}R^{14C}; or C(NH)NR^{13C}R^{14C}; and
R^{13C} and R^{14C} are each independently H; C₁-C₄ alkyl; (C₁-C₃ alkoxy)C₂-C₄ alkyl; or (hydroxy)C₂-C₄ alkyl; and
pharmaceutically acceptable salts thereof.

Preferred cGMP PDE_{V} inhibitors include 1-[[3-(6,7-dihydro-1-methyl-7-oxo-3-propyl-1 H-pyrazolo[4,3-d]pyrimidin-5-yl)-4-ethoxy-phenyl]sufonyl]-4-methylpiperazine (sildenafil) which has the structure of formula (VIII): 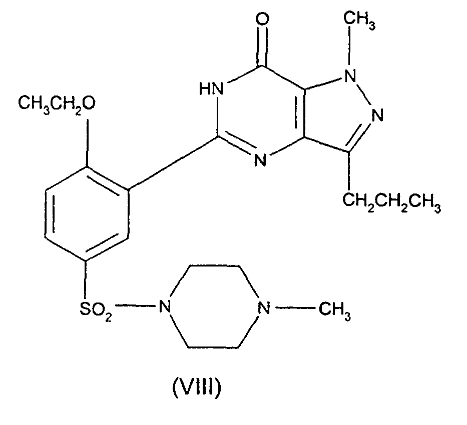 and pharmaceutically acceptable salts thereof; the compound having the structure of formula (IX): 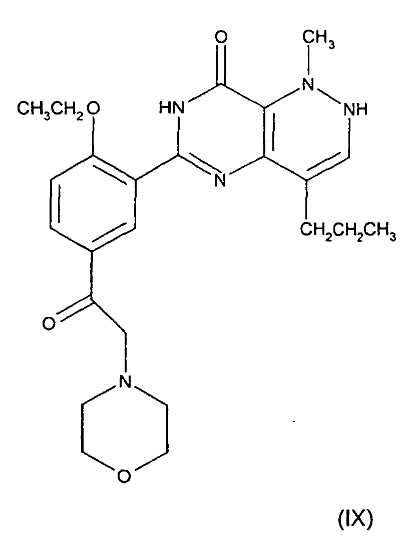 and pharmaceutically acceptable salts thereof; and the compound 3-ethyl-5-{5-[(4-ethylpiperazino) sulphonyl]-2-(2-methoxyethoxy)pyrid-3-yl}-2-(2-pyridylmethyl)-6,7-dihydro-2H-pyrazolo[4,3-d]pyrimidin-7-one of formula (X):

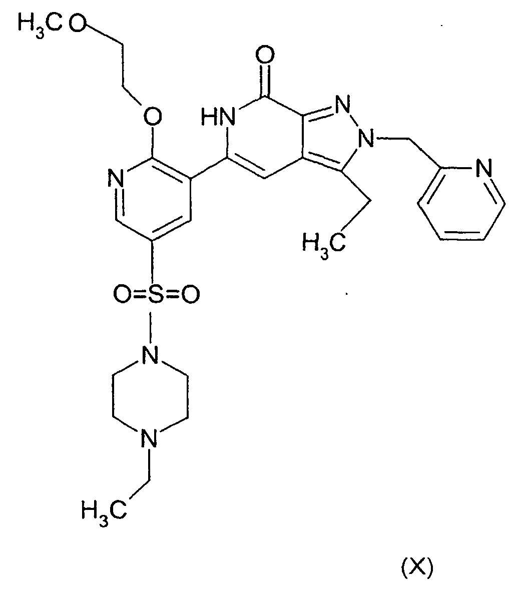

The compound of formula (IX) is disclosed, for example, in US Patents 5,272,147 and 5,426,107.

A preferred pharmaceutically acceptable salt of sildenafil for use in this invention is the citrate salt, and a preferred dosage range is from about 1 mg to about 100 mg.

Also preferred as cGMP PDEᵥ inhibitors are compounds disclosed in WO 95/19978 having the formula (XI): 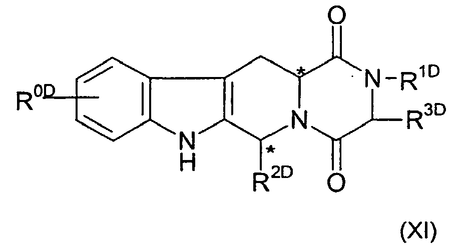 and salts and solvates thereof, in which:
R^{0D} represents hydrogen, halogen or C₁-C₆alkyl,;
R^{1D} represents hydrogen, C₁-C₆alkyl, C₂-C₆alkenyl, C₂-C₆alkynyl, haloC₁-C₆alkyl, C₃-C₈cydoalkyl, C₃-C₈cycloalkylC₁-C₃alkyl, arylC₁-C₃alkyl or heteroarylC₁-C₃alkyl;
R^{2D} represents an optionally substituted monocyclic aromatic ring selected from benzene, thiophene, furan and pyridine or an optionally substituted bicyclic ring 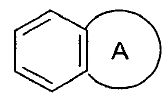 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen; and R^{3D} represents hydrogen or C₁-C₃alkyl, or R^{1D} and R^{3D} together represent a 3- or 4-membered alkyl or alkenyl ring.

A preferred group of compounds having formula Xla (also disclosed in WO 95/19978) includes compounds of the formula: 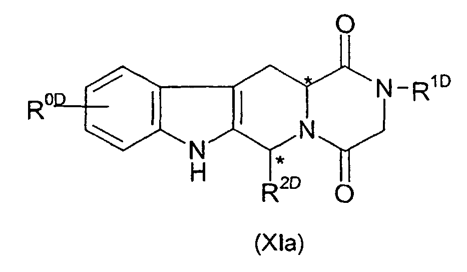 and salts and solvates thereof, in which:
R^{0D} represents hydrogen, halogen or C₁-C₆alkyl;
R^{1D} represents hydrogen, C₁-C₆alkyl, haloC₁-C₆alkyl, C₃-C₈cydoalkyl,
C₃-C₈cycloalkyl-C₁-C₃alkyl, arylC₁-C₃alkyl or heteroarylC₁-C₃alkyl; and
R^{2D} represents an optionally substituted monocyclic aromatic ring selected from benzene, thiophene, furan and pyridine or an optionally substituted bicyclic ring 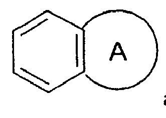 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen.

cGMP elevators of the present invention include produgs, geometric isomers, stereoisomers, hydrates, tautomers and salts of the described compounds.

Suitable cGMP PDE inhibitors also include those disclosed in the following US patents:
a 5-substituted pyrazolo[4,3-d]pyrimidine-7-one disclosed in US 4,666,908;
a griseolic acid derivative disclosed in any of US 4,634,706, 4,783,532, 5,498,819, 5,532,369, 5,556,975, and 5,616,600;
a 2-phenylpurinone derivative disclosed in US 4,885,301;
a phenylpyridone derivative disclosed in US 5,254,571;
a fused pyrimidine derivative disclosed in US 5,047,404;
a condensed pyrimidine derivative disclosed in US 5,075,310;
a pyrimidopyrimidine derivative disclosed in US 5,162,316;
a purine compound disclosed in US 5,073,559;
a quinazoline derivative disclosed in US 5,147,875;
a phenylpyrimidone derivative disclosed in US 5,118,686;
an imidazoquinoxalinone derivative or its aza analog disclosed in US 5,055,465 and 5,166,344;
a phenylpyrimidone derivative disclosed in US 5,290,933;
a 4-aminoquinazoline derivative disclosed in US 5,436,233 or 5,439,895;
a 4,5-dihydro-4-oxo-pyrrolo[1,2-a]quinoxaline derivative disclosed in US 5,405,847;
a polycyclic guanine derivative disclosed in US 5,393,755;
a nitogenous heterocyclic compound disclosed in US 5,576,322;
a quinazoline derivative disclosed in US 4,060,615;
a 6-heterocyclyl pyrazolo[3,4-d]pyrimidin-4-one disclosed in US 5,294,612; and
a 4-aminoquinazoline derivative disclosed in US 5,436,233;

Other disclosures of cGMP PDE inhibitors include:
European patent application (EPA) publication no. 0428268;
European patent 0442204;
International patent application publication no. WO 94/19351;
Japanese patent application 5-222000;
European Journal of Pharmacology, 251, (1994), 1;
International patent application publication no. WO 94/22855;
a pyrazolopyrimidine derivative disclosed in European patent application 0636626;
a 4-aminopyrimidine derivative disclosed in European patent application 0640599;
an imidazoquinazoline derivative disclosed in International patent application WO95/06648;
an anthranilic acid derivative disclosed in International patent application WO95/18097;
a tetracyclic derivative disclosed in International patent application WO95/19978;
an imidazoquinazoline derivative as disclosed in European patent application 0668280; and
a quinazoline compound disclosed in European patent application 0669324.

The cGMP PDE inhibition of a compound can be determined by standard assays known to the art, for example as disclosed in US 5,250,534. Compounds that are selective inhibitors of cGMP PDE relative to cAMP PDE are preferred, and determination of such compounds is also taught in US 5,250,534. Particularly preferred are compounds that selectively inhibit the PDEᵥ isoenzyme, as disclosed in WO 94/28902.

In addition, an estrogen agonist / antagonist can be used in combination with antibiotics such as azithromycin and/or antifungals such as fluconazole and vorconazole. Such combinations are particularly useful for treating vaginal or urinary bacterial or yeast infections.

The additional compounds that can be administered with an estrogen agonist / antagonist can be administered in the same dosage form or in different dosage forms. Likewise, the additional compounds can be administered at the same time as the estrogen agonist /antagonist or at different times. All combinations of dosage form and times are contemplated.

It is also noted that an estrogen agonist / antagonist can be administered using a topical dosage form such as a patch that is placed on the skin or an ointment that is rubbed on the skin. Such a dosage form can be used to administer other compounds that can be used in combination with an estrogen agonist / antagonist. In one embodiment, the estrogen agonist / antagonist is administered in combination with one or more additional compounds. In another embodiment, the additional compounds are administered using a topical dosage form and the estrogen agonist / antagonist is administered using a different dosage form such as a tablet.

The methods of measuring vaginal health involve an evaluation of vaginal health symptoms and a physical examination to determine physiological markers of vaginal health. The methods may also include a measurement of vaginal and/or urinary tract infection frequency and/or frequency of urinary incontinence. The evaluation of pathological vaginal symptoms and subject-perceived vaginal health status is self-evaluated by the subject.

Measurements that are made during a gynecological examination include a vaginal pH measurement and a determination of vaginal maturation index. Vaginal pH measurements are performed first during an examination and always prior to vaginal maturation index measurement or a PAP smear. If, at the beginning of the examination, a significant amount of lubricant is required to insert the speculum, the pH may be measured prior to the insertion of the speculum.

Vaginal pH measurements may be made with a single use, disposable pH probe such as the pHem-Alert^{™} pH probe (Imagyn Medical Technologies, Inc., Costa Mesa, CA). The pH probe is removed from packaging, inserted into the outer 1/3 of the vagina, and held against the lateral sidewall for about 2 seconds to moisten the paper. Contact between the pH probe and blood or cervical mucous should be avoided as the pH of these substances is generally neutral. The pH probe is then removed and immediately compared to the pH color chart accompanying the pH probe to determine the pH of the vagina.

The vaginal maturation index represents the degree of proliferation and maturation of vaginal cells. The results are reported as percentages of parabasal, intermediate, and superficial cells as determined by techniques known in the art. The vaginal maturation index smear must be taken prior to the PAP smear. A commercially available kit for cytological specimen collection may be used (PAP Pak^{™}, Medical Packaging Corp., Camarillo, CA) to obtain the maturation index specimen. Generally, a rounded spatula end is used to take a gentle scraping from the mid-third lateral area of the vagina and applied to a microscope slide. The sample must not be taken from the cervix or any other area of the vagina. The sample must also be collected from a healthy area free from inflammation, infection, ulceration, debris, or other contaminants. The preparation is immediately fixed by flooding the entire slide with standard cytology fixative. The fixative may be a standard fixative for cytology specimens such as that included in the PAP Pak^{™} specimen collection kit. Once the fixative has dried, the specimen is evaluated microscopically and the maturation index determined.

The subject self-assessment of vaginal health is performed with a subjective vaginal health questionnaire. Typically, the questionnaire is performed in private and the results kept confidential. The questionnaire may include an envelope that is specially encoded to later identify the treatment that the subject is undergoing. Examples of the questions and the ratings for questions are shown in Table 1. An additional question may be added in subsequent questionnaires following administration of the baseline questionnaire, which question would ask: "Have you had any positive or negative vaginal changes since you have been taking the study medication?" This question may be coupled to a multiple-point ratings scale ranging from "mostly positive changes," to "moderately positive changes," to "mildly positive changes," to "no changes," to "mildly negative changes," to "moderately negative changes," to "mostly negative changes."

**Table 1 : Subjective Vaginal Health Questionnaire Questions**

| | Question | Select Response or Report Number |
|---|---|---|
| 1 | How many children have you had by: | Natural Delivery? |
| | | Cesarean Section? |
| 2 | How many vaginal infections have you had in the last 6 months? | |
| 3 | How many urinary tract infections have you had in the last 6 months? | |
| 4 | What is the severity of any urinary incontinence that you experience? | a) No Leakage (never use protection)? |
| | | b) Mild Leakage (no protection necessary)? |
| | | c) Moderate Leakage (occasionally wear protection)? |
| | | d) Moderately Severe Leakage (regularly wear protection)? |
| | | e) Severe Leakage (must change protection more than 2 times per day)? |
| 5 | If you experience any amount of urinary incontinence, when does it occur? | a) When laughing, coughing, straining or physically active? |
| | | b) Leakage is uncontrollable and is preceded by a strong urge or need to urinate. |
| | | c) Leakage occurs without apparent cause. |
| 6 | What is the number that most closely represents your degree of vaginal dryness? | Scale of 1 to 7 with 1 representing no vaginal dryness and 7 representing extreme vaginal dryness. |
| 7 | What is the number that most closely represents your degree of vaginal itching or irritation? | Scale of 1 to 7 with 1 representing no vaginal itching or irritation and 7 representing extreme vaginal itching or irritation. |
| 8 | Are you taking any products to assist with vaginal dryness, irritation or itching? | |
| 9 | What is your overall vaginal health. | No Problems. |
| | | Mild Problems. |
| | | Moderate Problems. |
| | | Moderately Severe Problems. |
| | | Severe Problems. |
| 10 | What is the occurrence and frequency of any type of sexual activity? | a) None |
| | | b) Rare (less than 1 per month) |
| | | c) Infrequent (1-3 times per month) |
| | | d) Regular (1-3 times per week) |
| | | e) Frequent (more than 3 times per week) |
| 11 | What is the occurrence of orgasm (by any means) with sexual activity? | a) None |
| | | b) Rare (less than 1 per month) |
| | | c) Infrequent (1-3 times per month) |
| | | d) Regular (1-3 times per week) |
| | | e) Frequent (more than 3 times per week) |
| 12 | Has anything happened in your personal life to decrease your interest in sexual activity? | (e.g., change in health status or change in relationships, etc.) |

When the present assessment of vaginal health methods are used to evaluate drug efficacy or efficacy of other therapeutic methods, a vaginal health evaluation is made at the beginning of treatment. An additional evaluation is made one or more times during the course of or at the end of treatment such as every six months during the course of the study.

An important aspect of the present invention is the standardized assessment of pelvic organ prolapse and pelvic floor dysfunction. The present method can use a standardized system of terminology and grading from Baden, W. and Walter, T., Surgical Repair of Vaginal Defects, Philadelphia, JB Linnincott, 1992 and Bump, R.C. et al., The standardization of terminology of female pelvic organ prolapse and pelvic floor dysfunction, Am. J. Obstet. Gynecol. 175:10-17, 1996.

Exam variables are controlled so that accurate measurements may be made over the period of observation that may range anywhere from 6 months to 2 years or more. It is important, therefore, that certain examination variables be held constant for the duration such as: (i) position of the subject during examination; (ii) type of exam table or chair; (iii) type of specula or retractors; (iv) type of straining (Valsalva maneuver or cough); (v) fullness of bladder (void prior to exam); and (vi) content of rectum (is stool present on rectal exam?).

Vaginal prolapse is graded on a 0 to 4 scale for the following types of prolapse: anterior wall urethrocele; anterior wall cystocele; superior wall uterine prolapse, posterior wall enterocele; and posterior wall rectocele. All prolapses are assigned a grade as determined in Table 2.

**Table 2: Conditions Corresponding to Prolapse Grade**

| Grade | Criteria for Assigning Grade |
|---|---|
| Grade 0 | Normal position inside the mid-vaginal axis for anterior posterior wall prolapse and above the ischial spines for cervical or vaginal cuff. By definition, the most apical point is -3 cm superior to the hymen. |
| Grade 1 | If the prolapse crosses the respective thresholds, and descends halfway to the hymen. |
| Grade 2 | Descent to the hymen. |
| Grade 3 | Descent 2 cm beyond the hymen. |
| Grade 4 | Maximum possible descent for each site. A complete eversion is -5 cm beyond the hymen. |

For quantitation of pelvic organ position and prolapse, the following conditions apply: (i) the prolapse must be evaluated relative to a fixed point of reference; (ii) the ischial spines are reference for cervix or vaginal cuff prolapse; (iii) the vaginal midline axis will serve as a landmark for anterior and posterior wall prolapse; and (iv) the hymen will be the landmark for any prolapse extending beyond the ischial spines or the vaginal midline axis. The procedure for visualization of the prolapse is as follows:
(1) If an enterocele is suspected or the grade is uncertain, the examination is performed while the subject is standing.
(2) A posterior speculum blade or fingers are inserted to the vaginal apex, perineum is depressed, strain elicited, and withdrawal accomplished slowly to observe for prolapse and grade.
(3) The procedure is repeated by moving the speculum anteriorly to grade enterocele and rectocele if enterocele and rectocele are inadequately visualized in (2) above.
Finally, external urogenital measurements are made in centimeters. As the propensity of pelvic prolapse increases as the ratio of genital hiatus to perineal body measurement increases, the genital hiatus and perineal body are measured as follows:
Genital hiatus: Distance from the middle of the external meatus to the posterior midline hymen.
Perineal body: Distance from the posterior margin of the genital hiatus to the mid-anal opening.

During the examination for pelvic organ prolapse and pelvic floor dysfunction, objective measurements may also be made to evaluate the subject's vaginal health. These measurements may include measurements of blood estrogen and testosterone levels, measurement of vaginal pH, and measurement of the vaginal maturation index.

An internal vaginal health evaluation may be performed with continuously variable scale used under the following guidelines: The examiner is presented with a printed line of between 3 and 20 centimeters in length, preferably between 6 and 15 centimeters in length and most preferably 10 centimeters in length. The line is scaled with axis markers corresponding to degrees of internal vaginal health. For example, on a horizontal line, 10 centimeters in length, one end of the line may be marked with the numeral (I), the midpoint of the line marked with the numeral (II) and the end of the line opposite of numeral (I) is marked with the numeral (III). The numerals I-III are indicated to correspond to the following condition:
(I) No rugae, elasticity non-existent, friable and bleeds to the touch, mucosa very pale, very dry, stenotic, narrow vaginal apex.
(II) Few rugae, mild elasticity, mucosa pale in color (not pink), loss of some moisture, slightly stenotic, mild narrowing of vaginal apex.
(III) Rugae present, good elasticity, pink, roboust mucosa, good vaginal moisture, normal vaginal apex with good depth.
The examiner is instructed to place a mark on the line representing the continuum of vaginal conditions from numerals I-III that best describes the internal evaluation.

In addition to the internal evaluation, an external evaluation may be obtained describing, for example, the appearance of pubic hair (i.e. very scant, scant, moderate, normal, excessive) and the condition of the labia (i.e. full thickness, mild vulvar regression, moderate vulvar regression (atrophic labia) or labial fusion (severe atrophy)).

The data obtained from the vaginal examination and tests described above are used in their totality to assess the vaginal health of a patient. The methods can be used to determine if a compound that has been administered to a patient has affected vaginal health, or the methods can be used to help a clinician assess vaginal health for the purpose of making a diagnosis. The present methods can also be used to assess changes in vaginal heath over time.

Interestingly, through the use of a self-assessment questionnaire, it has been found that postmenopausal women taking the estrogen agonist/antagonist (-)-cis-6-phenyl-5-[4-(2-pyrrolidin-1-yl-ethoxy)-phenyl]-5,6,7,8-tetrahydro-naphthalene-2-ol, D-tartrate salt reported an increased frequency of sexual intercourse and/or an increased number of orgasms and/or increased intensity of orgasms.

The examples set forth below are intended to illustrate specific embodiments of the present invention and are not intended to limit the scope of the specification, including the claims, in any manner.

### EXAMPLES

### Example 1: Improvement or Maintenance of Urogenital Health.

Effects of estrogen agonists / antagonists for improving or maintaining urogenital health are assessed in a patient population of postmenopausal women not undergoing hormone replacement therapy. The efficacy of the estrogen agonist/antagonist for improving or maintaining urogenital health is measured in a random, double-blind, placebo controlled clinical study.

Patients are randomly separated into either a treatment group or a placebo group. Patients are initially given a subjective vaginal health questionnaire before receiving placebo or estrogen agonist / antagonist. Treatment or placebo is initiated and continued for 6 months. The questionnaire is administered to all patients at 3 and 6 months.

The patient self-assessment of vaginal health is performed with a subjective vaginal health questionnaire. The questionnaire is performed in private and the results kept confidential. The questionnaire is specially encoded to later identify the treatment that the patient is undergoing. Examples of the questions are given in Table 1. An additional question is added in subsequent questionnaires following the baseline questionnaire which would ask: "Have you had any positive or negative vaginal changes since you have been taking the study medication?" This question is coupled to a multiple-point ratings scale ranging from "mostly positive changes," to "moderately positive changes," to "mildly positive changes," to "no changes," to "mildly negative changes," to "moderately negative changes," to "mostly negative changes."

**Table 1 : Subjective Vaginal Health Questionnaire Questions**

| | Question | Select Response |
|---|---|---|
| 1 | How many children have you had by: | Natural Delivery? |
| | | Cesarean Section? |
| 2 | How many vaginal infections have you had in the last 6 months? | |
| 3 | How many urinary tract infections have you had in the last 6 months? | |
| 4 | What is the severity of any urinary incontinence that you experience? | a) No Leakage (never use protection)? |
| | | b) Mild Leakage (no protection necessary)? |
| | | c) Moderate Leakage (occasionally wear protection)? |
| | | d) Moderately Severe Leakage (regularly wear protection)? |
| | | e) Severe Leakage (must change protection more than 2 times per day)? |
| 5 | If you experience any amount of urinary incontinence, when does it occur? | a) When laughing, coughing, straining or physically active? |
| | | b) Leakage is uncontrollable and is preceded by a strong urge or need to urinate. |
| | | c) Leakage occurs without apparent cause. |
| 6 | What is the number that most closely represents your degree of vaginal dryness? | Scale of 1 to 7 with 1 representing no vaginal dryness and 7 representing extreme vaginal dryness. |
| 7 | What is the number that most closely represents your degree of vaginal itching or irritation? | Scale of 1 to 7 with 1 representing no vaginal itching or irritation and 7 representing extreme vaginal itching or irritation. |
| 8 | Are you taking any products to assist with vaginal dryness, irritation or itching? | |
| 9 | What is your overall vaginal health. | No Problems. |
| | | Mild Problems. |
| | | Moderate Problems. |
| | | Moderately Severe Problems. |
| | | Severe Problems. |
| 10 | What is the occurrence and frequency of any type of sexual activity? | a) None |
| | | b) Rare (less than 1 per month) |
| | | c) Infrequent (1-3 times per month) |
| | | d) Regular (1-3 times per week) |
| | | e) Frequent (more than 3 times per week) |
| 11 | What is the occurrence of orgasm (by any means) with sexual activity? | a) None |
| | | b) Rare (less than 1 per month) |
| | | c) Infrequent (1-3 times per month) |
| | | d) Regular (1-3 times per week) |
| | | e) Frequent (more than 3 times per week) |
| 12 | Has anything happened in your personal life to decrease your interest in sexual activity? | (e.g., change in health status or change in relationships, etc.) |

## Claims

1. The use of an estrogen agonist/antagonist of formula (I): 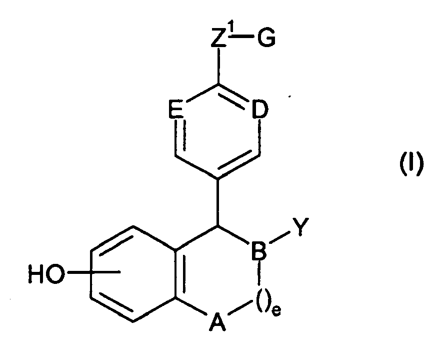 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ-, optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚW(CH₂)_{q}-;
(b) -O(CH₂)ₚCR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 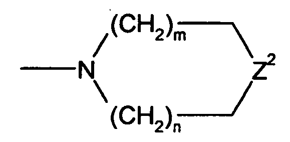 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 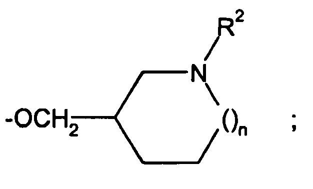
W is
(a) -CH₂-:
(b) -CH=CH-;
(c) -O-;
(d) -NR²-;
(e) -S(O)ₙ-;
(f) 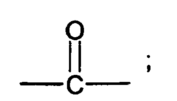
(g) -CR²(OH)-;
(h) -CONR²-;
(i) -NR²CO-;
(j) 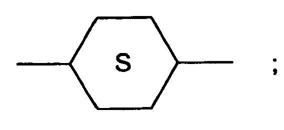 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxyl (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, or quaternary ammonium salt thereof, for the preparation of a medicament for treating vaginitis; treating bacterial vaginitis; treating a vaginal yeast or bacterial infection; treating urethrocele, cystocele, rectocele, or enterocele prolapse; or treating undesired urinary frequency or urgency; in a female patient.

2. The non-medical use of an estrogen agonist/antagonist of formula (I): 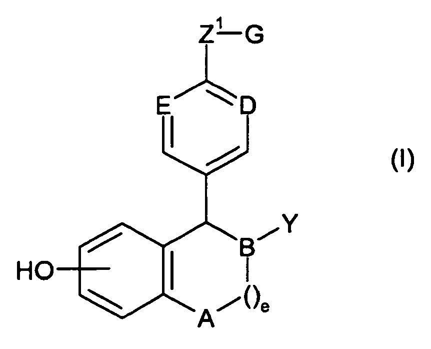 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ-, optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚW(CH₂)_{q}-;
(b) -O(CH₂)ₚCR⁵R⁶-;
(C) -O(CH₂)ₚW(CH₂)_{q}-;
(d) -OCHR²CHR³-; or
(e) -SCHR²CHR³-;
G is
(a) -NR⁷R⁸;
(b) 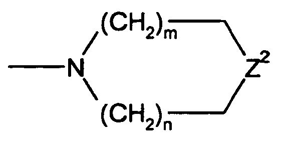 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 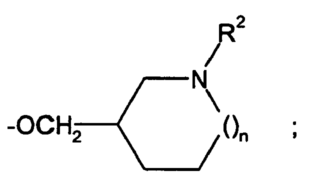
W is
(a) -CH₂-:
(b) -CH=CH-;
(c) -O-;
(d) -NR²-;
(e) -S(O)ₙ-;
(f) 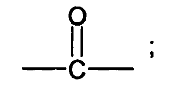
(g) -CR²(OH)-;
(h) -CONR²-;
(i) -NR²CO-;
(j) 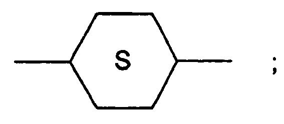 or
(k) -C=C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxyl (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester or quaternary ammonium salt thereof, for increasing the frequency or intensity of orgasms in a female patient.

3. The use of claim 1 or claim 2 wherein the estrogen agonist/antagonist is a compound of formula (IA) 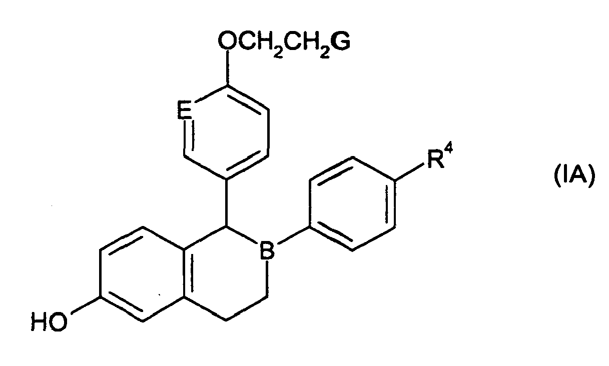 wherein G is 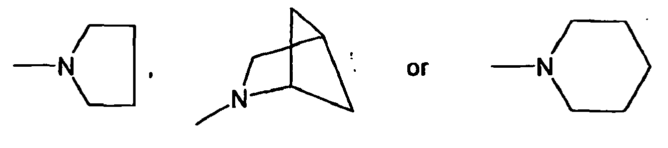 R⁴ is H, OH, F, or Cl; and B and E are independently selected from CH and N or an optical or geometric isomer thereof; or a pharmaceutical acceptable salt, N-oxide, ester, or quaternary ammonium salt, thereof.

4. The use of claim 1 or claim 2 wherein the estrogen agonist/antagonist is (-)-cis-6-phenyl-5-[4-(2-pyrrolidin-1-yl-ethoxy)-phenyl]-5,6,7,8-tetrahydronaphthalene-2-ol or an optical or geometric isomer thereof; a pharmaceutically acceptable salt, N-oxide, ester, or quaternary ammonium salt, thereof.

5. The use of claim 4 wherein the estrogen agonist/antagonist is in the form of a D-tartrate salt.

6. The use of claim 1 or claims 2 wherein the patient is a postmenopausal woman.

## Patentansprüche

1. Verwendung eines Östrogenagonisten/-antagonisten der Formel (I) : 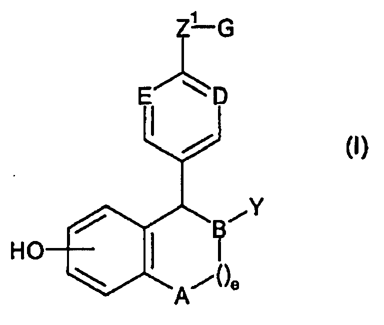 worin:
A ausgewählt ist aus CH₂ und NR;
B, D und E unabhängig ausgewählt sind aus CH und N;
Y
(a) Phenyl, gegebenenfalls substituiert mit 1-3 Substituenten, unabhängig ausgewählt aus R⁴;
(b) Naphthyl, gegebenenfalls substituiert mit 1-3 Substituenten, unabhängig ausgewählt aus R⁴;
(c) C₃-C₈-Cycloalkyl, gegebenenfalls substituiert mit 1-2 Substituenten, unabhängig ausgewählt aus R⁴;
(d) C₃-C₈-Cycloalkenyl, gegebenenfalls substituiert mit 1-2 Substituenten, unabhängig ausgewählt aus R⁴;
(e) ein fünfgliedriger Heterocyclus, der bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴;
(f) ein sechsgliedriger Heterocyclus, der bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴; oder
(g) ein bicyclisches Ringsystem, bestehend aus einem fünf- oder sechsgliedrigen heterocyclischen Ring, kondensiert an einen Phenylring, wobei der heterocyclische Ring bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴; ist;
Z₁
(a) -(CH₂)ₚW(CH₂)_{q}-,
(b) -O (CH₂)ₚCR⁵R⁶-,
(c) -O(CH₂)ₚW(CH₂)_{q}-,
(d) -OCHR²CHR³- oder
(e) -SCHR²CHR³-
ist;
G
(a) -NR⁷R⁸,
(b) 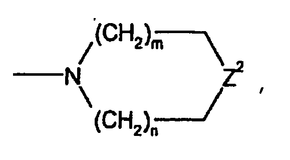 worin n 0, 1 oder 2 ist; m 1, 2 oder 3 ist; Z² -NH-, -O-, -S- oder -CH₂- ist; gegebenenfalls kondensiert an benachbarten Kohlenstoffatomen mit einem oder zwei Phenylringen und gegebenenfalls unabhängig substituiert am Kohlenstoff mit einem bis drei Substituenten und gegebenenfalls unabhängig substituiert am Stickstoff mit einem chemisch geeigneten Substituenten, ausgewählt aus R⁴; oder
(c) ein bicyclisches Amin, enthaltend fünf bis zwölf Kohlenstoffatome, entweder verbrückt oder kondensiert und gegebenenfalls substituiert mit 1-3 Substituenten, unabhängig ausgewählt aus R⁴;
ist; oder
Z¹ und G in Kombination 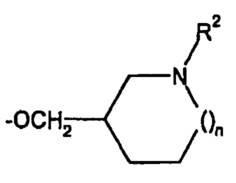 sein können;
W
(a) -CH₂-,
(b) -CH=CH-,
(c) -O-,
(d) -NR²-,
(e) -S(O)ₙ-,
(f) 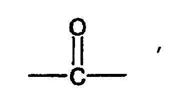
(g) -CR²(OH)-,
(h) -CONR²-,
(i) -NR²CO-,
(j) 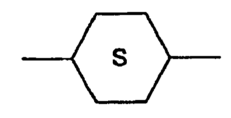 oder
(k) -C≡C-
ist;
R Wasserstoff oder C₁-C₆-Alkyl ist;
R² und R³ unabhängig
(a) Wasserstoff oder
(b) C₁-C₄-Alkyl
sind;
R⁴
(a) Wasserstoff,
(b) Halogen,
(c) C₁-C₆-Alkyl,
(d) C₁-C₄-Alkoxy,
(e) C₁-C₄-Acyloxy,
(f) C₁-C₄-Alkylthio,
(g) C₁-C₄-Alkylsulfinyl,
(h) C₁-C₄-Alkylsulfonyl,
(i) Hydroxyl(C₁-C₄)alkyl,
(j) Aryl(C₁-C₄)alkyl,
(k) -CO₂H,
(l) -CN,
(m) -CONHOR,
(n) -SO₂NHR,
(o) -NH₂,
(p) C₁-C₄-Alkylamino,
(q) C₁-C₄-Dialkylamino,
(r) -NHSO₂R,
(s) -NO₂,
(t) -Aryl oder
(u) -OH
ist;
R⁵ und R⁶ unabhängig C₁-C₈-Alkyl sind oder zusammen einen C₃-C₁₀-carbocyclischen Ring bilden;
R⁷ und R⁸ unabhängig
(a) Phenyl,
(b) ein C₃-C₁₀-carbocyclischer Ring, gesättigt oder ungesättigt,
(c) ein C₃-C₁₀-heterocyclischer Ring, enthaltend bis zu zwei Heteroatome, ausgewählt aus -O-, -N- und -S-,
(d) H,
(e) C₁-C₆-Alkyl sind oder
(f) einen 3- bis 8-gliedrigen Stickstoffenthaltenden Ring mit R⁵ oder R⁶ bilden;
R⁷ und R⁸ in entweder linearer oder Ringform gegebenenfalls mit bis zu drei Substituenten, unabhängig ausgewählt aus C₁-C₆-Alkyl, Halogen, Alkoxy, Hydroxy und Carboxy, substituiert sein können;
ein durch R⁷ und R⁸ gebildeter Ring gegebenenfalls an einen Phenylring kondensiert sein kann;
e 0, 1 oder 2 ist;
m 1, 2 oder 3 ist;
n 0, 1 oder 2 ist;
p 0, 1, 2 oder 3 ist;
q 0, 1, 2 oder 3 ist;
oder eines optischen oder geometrischen Isomers davon oder eines pharmazeutisch annehmbaren Salzes, N-Oxids, Esters oder quaternären Ammoniumsalzes davon zur Herstellung eines Medikamentes zum Behandeln von Vaginitis, Behandeln von bakterieller Vaginitis, Behandeln einer Hefe- oder bakteriellen Vaginainfektion, Behandeln von Urethrozele-, Zystozele-, Rektozele-, Enterozele-Prolaps oder Behandeln von unerwünschter Miktionshäufigkeit oder akutem Harndrang bei einer Patientin.

2. Nicht-medizinische Verwendung eines Östrogenagonisten/-antagonisten der Formel (I): 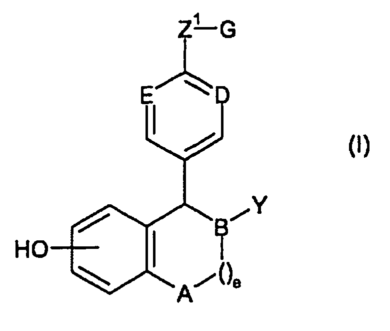 worin:
A ausgewählt ist aus CH₂ und NR;
B, D und E unabhängig ausgewählt sind aus CH und N;
Y
(a) Phenyl, gegebenenfalls substituiert mit 1-3 Substituenten, unabhängig ausgewählt aus R⁴;
(b) Naphthyl, gegebenenfalls substituiert mit 1-3 Substituenten, unabhängig ausgewählt aus R⁴;
(c) C₃-C₈-Cycloalkyl, gegebenenfalls substituiert mit 1-2 Substituenten, unabhängig ausgewählt aus R⁴;
(d) C₃-C₈-Cycloalkenyl, gegebenenfalls substituiert mit 1-2 Substituenten, unabhängig ausgewählt aus R⁴;
(e) ein fünfgliedriger Heterocyclus, der bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴;
(f) ein sechsgliedriger Heterocyclus, der bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴; oder
(g) ein bicyclisches Ringsystem, bestehend aus einem fünf- oder sechsgliedrigen heterocyclischen Ring, kondensiert an einen Phenylring, wobei der heterocyclische Ring bis zu zwei Heteroatome enthält, ausgewählt aus der Gruppe, bestehend aus -O-, -NR²- und -S(O)ₙ-, gegebenenfalls substituiert mit 1-3 Substituenten, unabhängig ausgewählt aus R⁴;
ist;
Z₁
(a) -(CH₂)ₚW(CH₂)_{q}-,
(b) -O (CH₂)ₚCR⁵R⁶-,
(c) -O(CH₂)ₚW(CH₂)_{q}-,
(d) -OCHR²CHR³- oder
(e) -SCHR²CHR³-
ist;
G
(a) -NR⁷R⁸,
(b) 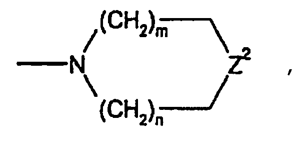 worin n 0, 1 oder 2 ist; m 1, 2 oder 3 ist; Z² -NH-, -O-, -S- oder -CH₂- ist; gegebenenfalls kondensiert an benachbarten Kohlenstoffatomen mit einem oder zwei Phenylringen und gegebenenfalls unabhängig substituiert am Kohlenstoff mit einem bis drei Substituenten und gegebenenfalls unabhängig substituiert am Stickstoff mit einem chemisch geeigneten Substituenten, ausgewählt aus R⁴; oder
(c) ein bicyclisches Amin, enthaltend fünf bis zwölf Kohlenstoffatome, entweder verbrückt oder kondensiert und gegebenenfalls substituiert mit 1-3 Substituenten, unabhängig ausgewählt aus R⁴;
ist; oder
Z¹ und G in Kombination 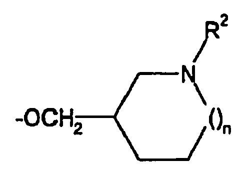 sein können;
W
(a) -CH₂-,
(b) -CH=C_{H}-,
(c) -O-,
(d) -NR²-,
(e) -S(O)ₙ-,
(f) 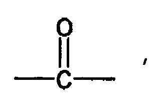
(g) -CR²(OH)-,
(h) -CONR²-,
(i) -NR²CO-,
(j) 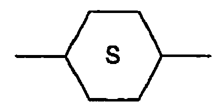 oder
(k) -C=C-
ist;
R Wasserstoff oder C₁-C₆-Alkyl ist;
R² und R³ unabhängig
(a) Wasserstoff oder
(b) C₁-C₄-Alkyl
sind;
R⁴
(a) Wasserstoff,
(b) Halogen,
(c) C₁-C₆-Alkyl,
(d) C₁-C₄-Alkoxy,
(e) C₁-C₄-Acyloxy,
(f) C₁-C₄-Alkylthio,
(g) C₁-C₄-Alkylsulfinyl,
(h) C₁-C₄-Alkylsulfonyl,
(i) Hydroxyl (C₁-C₄) alkyl,
(j) Aryl (C₁-C₄) alkyl,
(k) -CO₂H,
(l) -CN,
(m) -CONHOR,
(n) -SO₂NHR,
(o) -NH₂,
(p) C₁-C₄-Alkylamino,
(q) C₁-C₄-Dialkylamino,
(r) -NHSO₂R,
(s) -NO₂,
(t) -Aryl oder
(u) -OH
ist;
R⁵ und R⁶ unabhängig C₁-C₈-Alkyl sind oder zusammen einen C₃-C₁₀-carbocyclischen Ring bilden;
R⁷ und R⁸ unabhängig
(a) Phenyl,
(b) ein C₃-C₁₀-carbocyclischer Ring, gesättigt oder ungesättigt,
(c) ein C₃-C₁₀-heterocyclischer Ring, enthaltend bis zu zwei Heteroatome, ausgewählt aus -O-, -N- und -S-,
(d) H,
(e) C₁-C₆-Alkyl sind oder
(f) einen 3- bis 8-gliedrigen Stickstoffenthaltenden Ring mit R⁵ oder R⁶ bilden;
R⁷ und R⁸ in entweder linearer oder Ringform gegebenenfalls mit bis zu drei Substituenten, unabhängig ausgewählt aus C₁-C₆-Alkyl, Halogen, Alkoxy, Hydroxy und Carboxy, substituiert sein können;
ein durch R⁷ und R⁸ gebildeter Ring gegebenenfalls an einen Phenylring kondensiert sein kann;
e 0, 1 oder 2 ist;
m 1, 2 oder 3 ist;
n 0, 1 oder 2 ist;
p 0, 1, 2 oder 3 ist;
q 0, 1, 2 oder 3 ist;
oder eines optischen oder geometrischen Isomers davon oder eines pharmazeutisch annehmbaren Salzes, N-Oxids, Esters oder quaternären Ammoniumsalzes davon zur Erhöhung der Häufigkeit oder Intensität von Orgasmen bei einer Patientin.

3. Verwendung nach Anspruch 1 oder Anspruch 2, worin der Östrogenagonist/-antagonist eine Verbindung der Formel (IA) 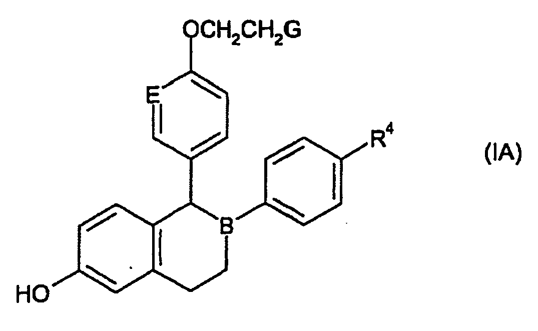 ist, worin G 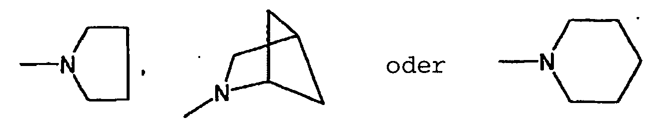 ist;
R⁴ H, OH, F oder Cl ist; und B und E unabhängig ausgewählt sind aus CH und N; oder eines optischen oder geometrischen Isomers davon oder eines pharmazeutisch annehmbaren Salzes, N-Oxids, Esters oder quaternären Ammoniumsalzes davon.

4. Verwendung nach Anspruch 1 oder 2, wobei der Östrogenagonist/-antagonist (-)-cis-6-Phenyl-5-[4-(2-pyrrolidin-1-ylethoxy)phenyl]-5,6,7,8-tetrahydronaphthalin-2-ol oder ein optisches oder geometrisches Isomer davon, ein pharmazeutisch annehmbares Salz, N-Oxid, ein pharmazeutisch annehmbarer Ester oder ein pharmazeutisch annehmbares quaternäres Ammoniumsalz davon ist.

5. Verwendung nach Anspruch 4, wobei der Östrogenagonist/-antagonist in der Form eines D-Tartratsalzes ist.

6. Verwendung nach Anspruch 1 oder Anspruch 2, wobei die Patientin eine Frau nach der Menopause ist.

## Revendications

1. Utilisation d'un agoniste/antagoniste des oestrogènes de formule (I) : 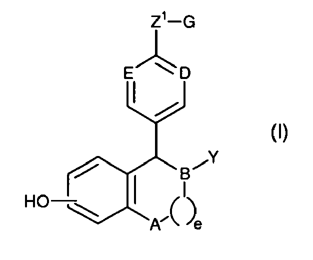 dans laquelle:
A est sélectionné parmi CH₂ et NR ;
B, D et E sont indépendamment sélectionnés parmi CH et N ;
Y représente
(a) phényle, facultativement substitué par 1-3 substituants indépendamment sélectionnés parmi les radicaux R⁴ ;
(b) naphtyle, facultativement substitué par 1-3 substituants indépendamment sélectionnés parmi les radicaux R⁴ ;
(c) cycloalkyle en C₃-C₈, facultativement substitué par 1-2 substituants indépendamment sélectionnés parmi les radicaux R⁴ ;
(d) cycloalkényle en C₃-C₈, facultativement substitué par 1-2 substituants indépendamment sélectionnés parmi les radicaux R⁴ ;
(e) un hétérocycle à cinq éléments contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ;
(f) un hétérocycle à six éléments contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ; ou
(g) un système de noyau bicyclique consistant en un noyau hétérocyclique à cinq ou six éléments condensé à un noyau phényle, ledit noyau hétérocyclique contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ;
Z¹ représente
(a) -(CH₂)ₚW(CH₂)_{q}- ;
(b) -O(CH₂)ₚCR⁵R⁶- ;
(c) -O(CH₂)ₚW(CH₂)_{q}- ;
(d) -OCHR²CHR³- ; ou
(e) -SCHR²CHR³- ;
G représente
(a) -NR⁷R⁸ ;
(b) 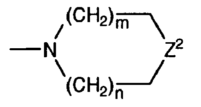 où n représente 0, 1 ou 2 ; m représente 1, 2 ou 3 ; Z² représente -NH-, -O-, -S- ou -CH₂- ; facultativement condensé sur des atomes de carbone adjacents avec un ou deux noyaux phényle et, facultativement et indépendamment substitué sur le carbone par de un à trois substituants et, facultativement et indépendamment, substitué sur l'azote par un substituant chimiquement convenable sélectionné parmi les radicaux R⁴ ; ou
(c) une amine bicyclique contenant de cinq à douze atomes de carbone, soit pontée soit condensée, et facultativement substituée par 1-3 substituants indépendamment sélectionnés parmi les radicaux R⁴ ; ou
Z¹ et G peuvent représenter, en combinaison : 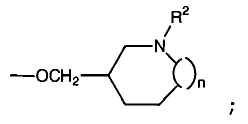
W représente :
(a) -CH₂- ;
(b) -CH=CH- ;
(c) -O- ;
(d) -NR²- ;
(e) -S(O)ₙ- ;
(f) 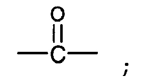
(g) -CR²(OH)- ;
(h) -CONR²- ;
(i) -NR²CO- ;
(j) 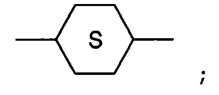 ou
(k) -C ≡ C- ;
R représente l'hydrogène ou alkyle en C₁-C₆ ;
R² et R³ représentent indépendamment
(a)l'hydrogène ; ou
(b) alkyle en C₁-C₄ ;
R⁴ représente :
(a) l'hydrogène ;
(b) un halogène ;
(c) alkyle en C₁-C₆ ;
(d) alcoxy en C₁-C₄ ;
(e) acyloxy en C₁-C₄ ;
(f) (alkyle en C₁-C₄)thio ;
(g) (alkyle en C₁-C₄)sulfinyle ;
(h) (alkyle en C₁-C₄)sulfonyle ;
(i) hydroxy(alkyle en C₁-C₄) ;
(j) (aryl)alkyle en C₁-C₄ ;
(k) -CO₂H ;
(l) -CN ;
(m) -CONHOR ;
(n) -SO₂NHR ;
(O) -NH₂ ;
(p) (alkyle en C₁-C₄)amino ;
(q) (dialkyle en C₁-C₄)amino ;
(r) -NHSO₂R ;
(s) -NO₂ ;
(t) -aryle ; ou
(u) -OH ;
R⁵ et R⁶ représentent indépendamment alkyle en C₁-C₈ ou forment ensemble un noyau carbocyclique en C₃-C₁₀ ;
R⁷ et R⁸ représentent indépendamment:
(a) phényle ;
(b) un noyau carbocyclique en C₃-C₁₀, saturé ou insaturé ;
(c) un noyau hétérocyclique en C₃-C₁₀ contenant jusqu'à deux hétéroatomes sélectionnés parmi -O-, -N- et -S- ;
(d) H ;
(e) alkyle en C₁-C₆ ; ou
(f) un noyau ayant de 3 à 8 éléments, contenant de l'azote, formé avec R⁵ ou R⁶ ;
R⁷ et R⁸, sous forme linéaire ou cyclique, peuvent être facultativement substitués par jusqu'à trois substituants indépendamment sélectionnés parmi les radicaux alkyle en C₁-C₆, halogéno, alcoxy, hydroxy et carboxy ;
un noyau formé par R⁷ et R⁸ peut facultativement être condensé à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;
ou un isomère optique ou géométrique d'un tel composé ; ou un sel, N-oxyde, ester ou sel d'ammonium quaternaire pharmaceutiquement acceptable d'un tel composé ; dans la préparation d'un médicament pour le traitement d'une vaginite ; le traitement d'une vaginite bactérienne ; le traitement d'une infection vaginale fongique ou bactérienne ; le traitement du prolapsus de type uréthrocèle, cystocèle, rectocèle ou entérocèle ; ou pour le traitement du caractère fréquent ou impérieux de la miction ; chez un patient de sexe féminin.

2. Utilisation non-médicale d'un agoniste/antagoniste des oestrogènes de formule (I) : 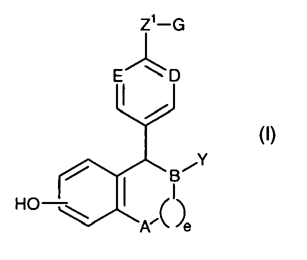 dans laquelle :
A est sélectionné parmi CH₂ et NR ;
B, D et E sont indépendamment sélectionnés parmi CH et N ;
Y représente
(a) phényle, facultativement substitué par 1-3 substituants indépendamment sélectionnés parmi les radicaux R⁴ ;
(b) naphtyle, facultativement substitué par 1-3 substituants indépendamment sélectionnés parmi les radicaux R⁴ ;
(c) cycloalkyle en C₃-C₈, facultativement substitué par 1-2 substituants indépendamment sélectionnés parmi les radicaux R⁴ ;
(d) cycloalkényle en C₃-C₈, facultativement substitué par 1-2 substituants indépendamment sélectionnés parmi les radicaux R⁴;
(e) un hétérocycle à cinq éléments contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ;
(f) un hétérocycle à six éléments contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ; ou
(g) un système de noyau bicyclique consistant en un noyau hétérocyclique à cinq ou six éléments condensé à un noyau phényle, ledit noyau hétérocyclique contenant jusqu'à deux hétéroatomes sélectionnés dans le groupe consistant en -O-, -NR²- et -S(O)ₙ-, facultativement substitué par 1-3 substituants indépendamment sélectionnés parmi les radicaux R⁴ ;
Z¹ représente
(a) -(CH₂)ₚW(CH₂)_{q}- ;
(b) -O(CH₂)ₚCR⁵R⁶- ;
(c) -O(CH₂)ₚW(CH₂)_{q}- ;
(d) -OCHR²CHR³- ; ou
(e) -SCHR²CHR³- ;
G représente
(a) -NR⁷R⁸ ;
(b) 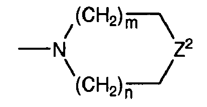 où n représente 0, 1 ou 2 ; m représente 1, 2 ou 3 ; Z² représente -NH-, -O-, -S- ou -CH₂- ; facultativement condensé sur des atomes de carbone adjacents avec un ou deux noyaux phényle et, facultativement et indépendamment substitué sur le carbone par de un à trois substituants et, facultativement et indépendamment, substitué sur l'azote par un substituant chimiquement convenable sélectionné parmi les radicaux R⁴ ; ou
(c) une amine bicyclique contenant de cinq à douze atomes de carbone, soit pontée soit condensée, et facultativement substituée par 1-3 substituants indépendamment sélectionnés parmi les radicaux R⁴ ; ou
Z¹ et G peuvent représenter, en combinaison : 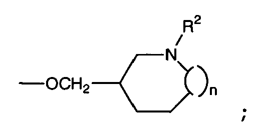
W représente :
(a) -CH₂- ;
(b) -CH=CH- ;
(c) -0- ;
(d) -NR²- ;
(e) -S(O)ₙ- ;
(f) 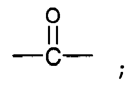
(g) -CR²(OH)- ;
(h) -CONR²- ;
(i) -NR²CO- ;
(j) 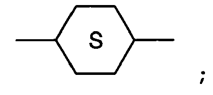 ou
(k) -C ≡ C- ;
R représente l'hydrogène ou alkyle en C₁-C₆ ;
R² et R³ représentent indépendamment
(a) l'hydrogène ; ou
(b) alkyle en C₁-C₄ ;
R⁴ représente :
(a) l'hydrogène ;
(b) un halogène ;
(c) alkyle en C₁-C₆ ;
(d) alcoxy en C₁-C₄ ;
(e) acyloxy en C₁-C₄ ;
(f) (alkyle en C₁-C₄)thio ;
(g) (alkyle en C₁-C₄)sulfinyle ;
(h) (alkyle en C₁-C₄)sulfonyle ;
(i) hydroxy(alkyle en C₁-C₄) ;
(j) (aryl)alkyle en C₁-C₄ ;
(k) -CO₂H ;
(l) -CN ;
(m) -CONHOR ;
(n) -SO₂NHR ;
(o) -NH₂ ;
(p) (alkyle en C₁-C₄)amino ;
(q) (dialkyle en C₁-C₄)amino ;
(r) -NHSO₂R ;
(s) -NO₂ ;
(t) -aryle ; ou
(u) -OH ;
R⁵ et R⁶ représentent indépendamment alkyle en C₁-C₈ ou forment ensemble un noyau carbocyclique en C₃-C₁₀ ;
R⁷ et R⁸ représentent indépendamment:
(a) phényle ;
(b) un noyau carbocyclique en C₃-C₁₀, saturé ou insaturé ;
(c) un noyau hétérocyclique en C₃-C₁₀ contenant jusqu'à deux hétéroatomes sélectionnés parmi -O-, -N- et -S- ;
(d) H ;
(e) alkyle en C₁-C₆ ; ou
(f) un noyau ayant de 3 à 8 éléments, contenant de l'azote, formé avec R⁵ ou R⁶ ;
R⁷ et R⁸, sous forme linéaire ou cyclique, peuvent être facultativement substitués par jusqu'à trois substituants indépendamment sélectionnés parmi les radicaux alkyle en C₁-C₆, halogéno, alcoxy, hydroxy et carboxy ;
un noyau formé par R⁷ et R⁸ peut facultativement être condensé à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;
ou un isomère optique ou géométrique d'un tel composé ; ou un sel, N-oxyde, ester ou sel d'ammonium quaternaire pharmaceutiquement acceptable d'un tel composé, pour augmenter la fréquence ou l'intensité des orgasmes chez un patient de sexe féminin.

3. Utilisation selon la revendication 1 ou la revendication 2, dans laquelle l'agoniste/antagoniste des oestrogènes est un composé de formule (IA) 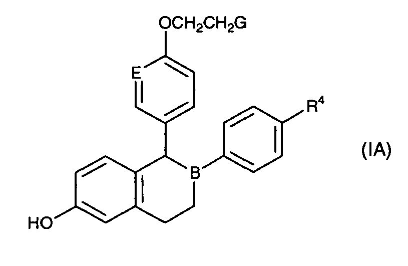 dans laquelle G représente 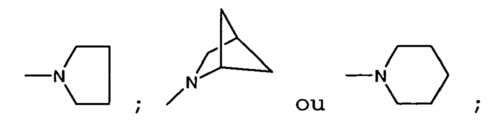 R⁴ représente H, OH, F ou Cl ; et B et E sont indépendamment sélectionnés parmi CH et N ou un isomère optique ou géométrique d'un tel composé ; ou un sel, N-oxyde, ester ou sel d'ammonium quaternaire pharmaceutiquement acceptable d'un tel composé.

4. Utilisation selon la revendication 1 ou la revendication 2, dans laquelle l'agoniste/antagoniste des oestrogènes est le (-)-cis-6-phényl-5-[4-(2-pyrrolidin-1-yl-éthoxy)-phényl]-5,6,7,8-tétrahydro-naphtalén-2-ol ou un isomère optique ou géométrique d'un tel composé ; ou un sel, N-oxyde, ester ou sel d'ammonium quaternaire pharmaceutiquement acceptable d'un tel composé.

5. Utilisation selon la revendication 4, dans laquelle l'agoniste/antagoniste des oestrogènes est sous la forme d'un D-tartrate.

6. Utilisation selon la revendication 1 ou la revendication 2, dans laquelle le patient est une femme post-ménopausée.
